# FASCICULE DE BREVET EUROPEEN

(11) **EP 0 789 750 B1**
(45) Date de publication et mention de la délivrance du brevet: **27.06.2001**
(21) Numéro de dépôt: 95936620.4
(22) Date de dépôt: 27.10.1995
(51) Int. Cl.: C12H 1/14, C12H 1/00, C12P 21/00

(54) **PRODUIT BIOLOGIQUE POUR LA STABILISATION PHYSICO-CHIMIQUE DES VINS**
BIOLOGISCHES PRODUKT ZUR PHYSIKALISCH-CHEMISCHEN WEINSTABILISIERUNG
BIOLOGICAL PRODUCT FOR THE PHYSICO-CHEMICAL STABILISATION OF WINE

(30) Priorité: 31.10.1994 FR 9413261
(43) Date de publication de la demande: 20.08.1997
(73) Titulaire: Faculté d'Oenologie, 33400 Talence (FR)
(72) Inventeur: MOINE, Virginie, F-33600 Pessac (FR); DUBOURDIEU, Denis, F-33410 Beguey (FR)
(74) Mandataire: Thébault, Jean-Louis
(86) Numéro de dépôt international: FR9501426
(87) Numéro de publication internationale: WO9613571

(56) Documents cités:
- APPLIED AND ENVIRONMENTAL MICROBIOLOGY., vol. 54, no. 6, WASHINGTON DC, US., pages 1420-1425, D.R. CAMERON ET AL. 'The mannoprotein of Saccharomyces cerevisiae is an effective bioemulsifier.'
- JOURNAL INTERNATIONAL DES SCIENCES DE LA VIGNE ET DU VIN., vol. 27, no. 1, FRANCE, pages 13-22, S. LUBBERS ET AL. 'Effet colloide-protecteur d'extraits de parois de levures sur la stabilité tartrique d'une solution hydro-alcoolique modele.'
- JOURNAL INTERNATIONAL DES SCIENCES DE LA VIGNE ET DU VIN., vol. 26, no. 4, FRANCE, pages 239-251, V. LEDOUX ET AL. 'Interprétation de l'amélioration de la stabilité protéique des vins au cours de l'élevage sur lies.'
- CARBOHYDRATE POLYMERS, vol. 23, no. 3, BARKING GB, pages 185-191, E.J. WATERS ET AL. 'A saccharomyces mannoprotein that protects wine from protein haze.'

## Description

La présente invention a pour objet un produit biologique pour le traitement du vin et plus particulièrement pour améliorer la stabilité protéique des vins blancs et rosés et pour éviter les précipitations tartriques des vins blancs, rosés et rouges.

On sait que la commercialisation des vins en bouteilles demande non seulement que le vin soit limpide lors de la mise en bouteille mais qu'il le reste au cours du temps, surtout pour les vins qui sont conservés sur des durées relativement longues.

Or on sait que les traitements actuels de stabilisation des vins sont peu satisfaisants, que ce soit face aux précipitations de sels tartriques ou protéiques dites dans le métier de l'oenologie "casses tartriques et protéiques".

En effet, pendant la conservation des vins blancs, la casse protéique conduit à des troubles ou des dépôts spécifiques qui apparaissent dans la bouteille lorsque la température de conservation du vin est élevée et/ou par suite de l'enrichissement du vin en tanins du bouchon.

Une solution connue consiste à traiter les moûts et les vins à la bentonite mais les doses nécessaires en fonction des besoins peuvent être suffisamment élevées pour appauvrir les caractéristiques organoleptiques des vins ainsi traités.

D'autres essais ont été conduits par voie enzymatique en utilisant des protéases exogènes ou à partir de levures afin d'éliminer les protéines responsables de la casse protéique mais les résultats n'ont pas donné satisfaction.

Il a été récemment montré par les inventeurs de la présente demande qu'au cours de l'élevage sur lies des vins blancs, l'amélioration constatée de la stabilité protéique des vins blancs était due à la présence de mannoprotéines libérées par les levures.

Quant à la précipitation des sels tartriques, présents essentiellement sous la forme de tartrate acide de potassium, ils sont en état de sursaturation dans les vins. Aussi, durant l'élevage du vin en hiver, le froid provoque les précipitations cristallines mais de toutes les façons cette précipitation survient également au cours du temps et sont visibles dans les bouteilles.

Des solutions pour stabiliser ces sels tartriques ont été recherchées et l'on en connaît actuellement trois.

La première solution consiste à hâter la précipitation par la conservation du vin pendant plusieurs semaines à des températures négatives puis à filtrer le vin pour en retirer les cristaux.

La deuxième solution consiste à améliorer la première en ajoutant au vin des doses élevées de cristaux si bien que le traitement par le froid est rendu plus efficace et sa durée plus courte, la filtration restant obligatoire pour retirer les cristaux supplémentaires et les cristaux formés.

La troisième solution consiste à ajouter dans le vin de l'acide métatartrique qui s'oppose à la cristallisation des sels tartriques sous les effets du froid. L'effet de protection disparaît dès lors que l'acide métatartrique s'hydrolyse, ce qui est d'autant plus rapide que la température de stockage du vin est élevée.

Ces solutions ne sont pas satisfaisantes car les deux premières sont longues et chères en plus de modifier les caractéristiques organoleptiques du vin traité.

Quant à la troisième, son efficacité est de courte durée donc réservée à des vins destinés à une consommation rapide, outre le fait qu'il faut introduire dans le vin un composé étranger initialement absent.

Des essais plus récents ont montré que les mannoprotéines extraites à la chaleur de parois de levures appartenant à l'espèce *Saccharomyces cerevisiae* ont un effet inhibiteur de la cristallisation des sels tartriques.

Le procédé d'obtention de ces mannoprotéines consiste à les solubiliser par la chaleur à 100° C en milieu aqueux et à les recueillir, soit directement par lyophilisation, soit par précipitation à l'éthanol et par séchage du précipité après centrifugation.

Cependant, l'efficacité des préparations proposées mises en évidence dans un milieu modèle n'est pas vérifiée dans la plupart des vins. En outre, cette préparation de mannoprotéines n'apporte aucune amélioration à l'instabilité protéique des vins blancs.

On connaît par la publication au nom de D.R.Cameron & al "the mannoprotein of Saccharomyces cerevisiae is an effective bioemulsifier" de la revue Applied and Environmental Microbiology, Vol. 54, No. 6, p. 1420-1425 (Juin 1988) un procédé d'obtention de mannoprotéines de levures obtenues par digestion enzymatique de levures entières au moyen de la zymolase ou zymolyase qui est uniquement une β 1-3 glucanase. Cette digestion enzymatique ne permet donc d'obtenir que des mannoprotéines à activités limitées.

On connaît aussi un article publié dans le Journal international des Sciences de la vigne et du vin Vol. 27, No. 1, p. 13-22 (1993), au nom de S.Lubbers & al sur le thème "Effet colloïde-protecteur d'extraits de parois de levures sur la stabilité tartrique d'une solution hydro-alcoolique modèle". Il s'agit là de mannoprotéines extraites à la chaleur qui ne permettent pas d'obtenir les effets de stabilisation recherchés. Les quantités obtenues ne peuvent être que pour des essais en modèle laboratoire sans possibilité de transfert sur le plan production industrielle.

D'autres publications peuvent être citées pour compléter l'art antérieur connu comme l'article publié dans le Journal international des Sciences de la vigne et du vin Vol. 26, No 4, p. 239-251 (1992) au nom de V.Ledoux & al sur le thème "Interprétation de l'amélioration de la stabilité protéique des vins au cours de l'élevage sur lies" et dans la revue Carbohydrate polylmers Vol. 23, p. 185-191 (1994), un article de E.J. Waters& al "A Saccharomyces mannoprotein that protects wine from protein haze"

Le but de la présente invention est de proposer un traitement des vins, blancs, rouges ou rosés afin de les stabiliser vis à vis des précipitations tartriques et protéiques, avec un effet suffisamment long pour être considéré comme permanent. De plus, le traitement prévoit l'ajout d'un produit biologique obtenu par un procédé selon l'invention, faisant intervenir des produits admis par la législation sur les vins, ce produit biologique étant inodore et parfaitement soluble dans les vins.

Selon la présente invention, le traitement d'un vin en vue de sa stabilisation vis à vis des sels tartriques et des protéines, est caractérisé en ce qu'il consiste à ajouter au vin des mannoprotéines extraites des parois de levures par digestion enzymatique.

Ce traitement se caractérise plus particulièrement en ce qu'il consiste à ajouter au vin des mannoprotéines extraites des parois de levure par digestion enzymatique par un mélange de β-1-3 et β-1-6 glucanases.

Selon l'invention, les levures utilisées appartiennent à l'espèce *Saccharomyces cerevisiae* et la quantité de mannoprotéines utilisée dans le vin est inférieure à 30 g/hl.

L'invention concerne également un procédé d'extraction de mannoprotéines par digestion enzymatique de levures pour la mise en oeuvre du traitement selon l'invention caractérisé en ce que :
- on fait incuber des parois de levure en milieu aqueux en présence de β-glucanases,
- on sépare les matières solides,
- on concentre la phase liquide, notamment par ultrafiltration,
- une étape supplémentaire consister à sécher le produit obtenu notamment par lyophilisation ou atomisation, par exemple dans un but de manipulation aisée.

Le procédé selon l'invention se caractérise en ce que les β-glucanases utilisées sont de type β-1-3 et du β-1-6 glucanases.

L'invention décrit également une mannoprotéine obtenue par ledit procédé pour la mise en oeuvre du procédé selon l'invention, caractérisée en ce que l'analyse par chromatographie liquide à haute pression de tamisage moléculaire montrent la présence d'un pic caractéristique, en ce que l'analyse par électrophorèse SDS PAGE montre la présence de deux protéines de 41 600 et 31 800 Daltons et en ce que l'analyse par électrophorèse capillaire montre la présence d'un pic caractéristique correspondant à la mannoprotéine de 31 800 Daltons.

Le traitement selon la présente invention est décrit ci-après ainsi qu'un mode de réalisation particulier du procédé d'extraction des mannoprotéines nécessaires à ce traitement.
- Figure 1 représente un chromatogramme par chromatographie liquide à haute pression de tamisage des mannoprotéines extraites par digestion enzymatique,
- Figure 2 représente un chromatogramme des mannoprotéines extraites par la chaleur,

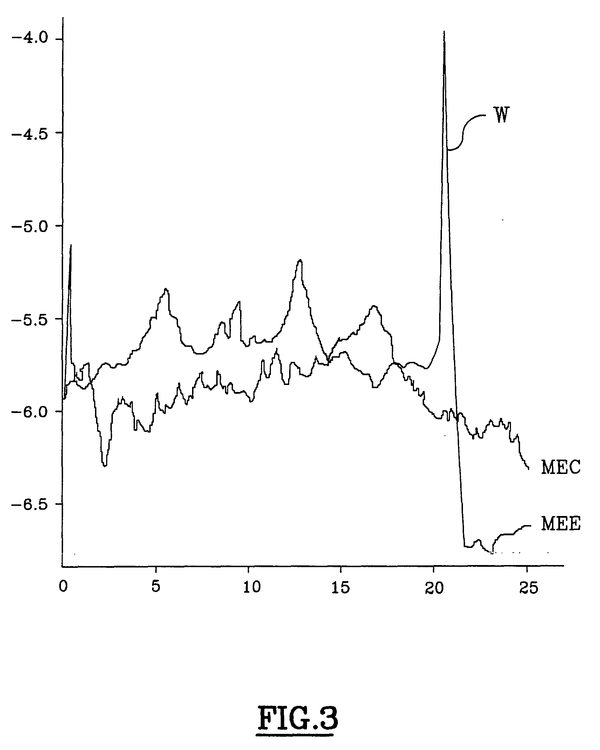

- Figure 3 représente le graphe obtenu par électrophorèse capillaire des mannoprotéines extraites par digestion enzymatique (MEE) comparé au graphe des mannoprotéines extraites par la chaleur (MEC),
- Figure 4 représente les fractions de protéines obtenues par élution au chlorure de sodium,
- Figure 5 représente l'indice de turbidité (NTU) en fonction des vins témoins et traités avec les différentes fractions,
- Figure 6 représente les fractions de protéines obtenues par élution avec de α-D-mannoside,
- Figure 7 représente l'indice de turbidité (NTU) en fonction des vins témoins et traités par les différentes fractions,
- Figure 8 est une comparaison de la concentration de MP 32 dans les différentes fractions,
- Figure 9 représente le pourcentage de MP32 dans les MEC et MEE.

Le procédé selon l'invention est maintenant décrit ci-après selon un mode de réalisation particulier.

Des parois de levures, plus particulièrement *Saccharomyces cerevisiae,* sont incubées à 40° C dans de l'eau, en présence d'une préparation de β-glucanase, notamment commercialisée par la société Novo sous la dénomination commerciale "*Glucanex®*".

Cette préparation comprend des activités exo-β-(1-3)-glucanase, endo-β-(1-3)-glucanase et exo-β-(1-6)-glucanase.

On sépare ensuite les matières solides.

La phase liquide est ensuite concentrée notamment par ultrafiltration.

Le produit en poids sec correspond sensiblement à 50 % du poids de parois cellulaires initialement introduites dans la préparation.

Le produit se compose de 88 % de polysaccharides, de 4 % de protéines et de 8 % d'indosé, sans doute le taux d'hydratation du produit.

Le produit est inodore, soluble dans l'eau et les vins et ne colmate pas les surfaces filtrantes utilisées pour la filtration des vins.

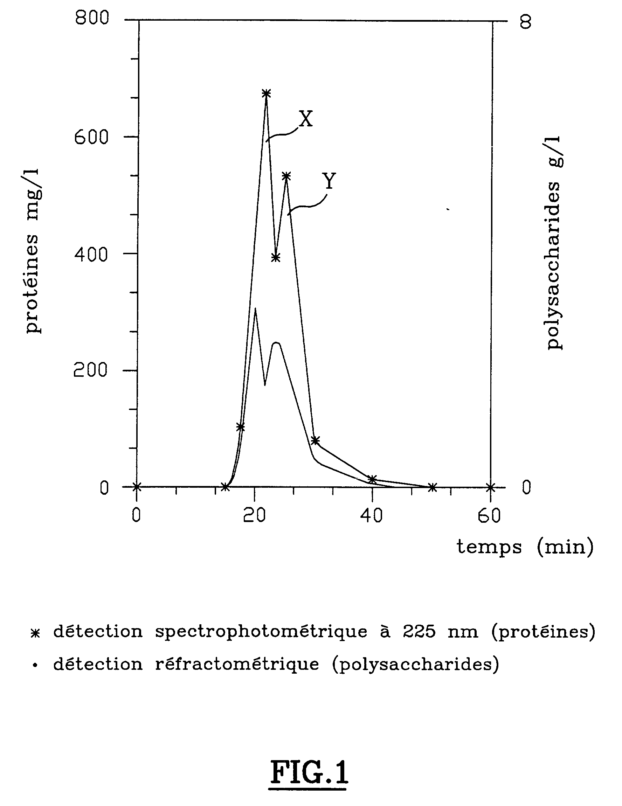

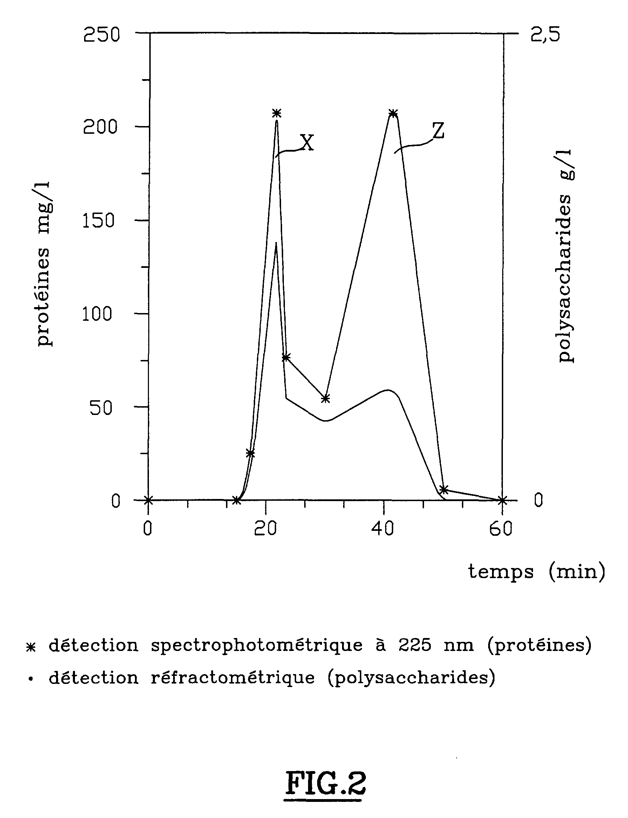

En se reportant aux figures 1 et 2, on remarque une différence notable entre les graphes qui sont tracés pour chacune de ces figures par détection spectrophotométrique à 225 nm pour les protéines et par détection réfractométrique pour les polysaccharides.

En effet, le graphe de la figure 2 présente un premier pic (X) correspondant au volume vide de la colonne et un second pic (Z) correspondant au volume total de la colonne.

On constate que le graphe de la figure 1 présente un second pic (Y) à proximité immédiate du pic (X) du volume vide de la colonne, absent du graphe de la figure 2.

Le procédé selon l'invention a permis l'extraction et la conservation d'une mannoprotéine particulière.

D'autre part, analysées en électrophorèse capillaire, en conditions non dénaturantes, sur une colonne de silice fondue, les mannoprotéines, extraites par digestion enzymatique, présentent un pic (W) correspondant à la mannoprotéine responsable de la thermostabilisation des protéines du vin blanc. Ce pic est en revanche absent lors de l'analyse des mannoprotéines extraites par la chaleur, toujours par électrophorèse capillaire.

On a ensuite procédé à des vérifications concernant les effets des mannoprotéines extraites par voie enzymatique à partir de différentes souches de levures appartenant toutes à l'espèce *Saccharomyces cerevisiae,* dites "MEE", vis à vis de la stabilisation tartrique, d'une part et vis à vis de la stabilisation protéique, d'autre part.

### I/ STABILISATION TARTRIQUE

On connaît deux types d'essais :

### 1.- Détermination de l'Indice de Stabilité Tartrique.

On introduit dans les échantillons à tester du bitartrate de potassium en quantités variables que l'on solubilise par chauffage à 30°C et puis on refroidit à - 4°C.

Plus le milieu est stable et plus la quantité de bitartrate de potassium nécessaire est importante pour provoquer la cristallisation.

L'appréciation de la cristallisation se fait visuellement ou par détermination par photométrie de flamme de la différence de concentration en potassium du vin filtré avant et après passage au froid.

Un vin est considéré comme stable si l'ajout de 75 mg de bitartrate de potassium pour 100 ml d'échantillon ne provoque pas de cristallisation.

### Essai en milieu modèle.

Les résultats des essais sont donnés ci-après, en les faisant porter sur 100 ml d'un milieu hydroalcoolique modèle composé de :
- 1,1 g/l de chlorure de potassium,
- 2,1 g/l d'acide tartrique, et
- 10,5 % d'éthanol.

Le paramètre variable est la quantité d'hydrogénotartrate de potassium, ajoutée en doses de : 0, 50, 75, 100 et 125 mg.

Les échantillons sont testés comparativement avec adjonction d'acide métatartrique, de mannoprotéines extraites par la chaleur (MEC) et de mannoprotéines extraites par digestion enzymatique (MEE) de trois sortes.

Les résultats du tableau ci-après rapportent les différences de concentration en potassium (mg/l) des échantillons avant et après passage au froid.

| | | | | | |
|---|---|---|---|---|---|
| THK mg/100 ml | 0 | 50 | 75 | 100 | 125 |
| Témoin | 0 | 0 | 40 | 100 | 180 |
| Ac méta 5 g/hl | 0 | 0 | 0 | 0 | 0 |
| Ac méta 10 g/hl | 0 | 0 | 0 | 0 | 0 |
| Ac méta 25 g/hl | 0 | 0 | 0 | 0 | 0 |
| MEC 10 g/hl | 0 | 0 | 30 | 100 | 180 |
| MEC 25 g/hl | 0 | 0 | 0 | 20 | 100 |
| MEC 50 g/hl | 0 | 0 | 0 | 0 | 40 |
| MEE1 10 g/hl | 0 | 0 | 0 | 60 | 120 |
| MEE1 25 g/hl | 0 | 0 | 0 | 60 | 100 |
| MEE1 50 g/hl | 0 | 0 | 0 | 40 | 100 |
| MEE2 10 g/hl | 0 | 0 | 60 | 80 | 120 |
| MEE2 25 g/hl | 0 | 0 | 0 | 0 | 60 |
| MEE2 50 g/hl | 0 | 0 | 0 | 0 | 0 |
| MEE3 10 g/hl | 0 | 0 | 80 | 120 | 180 |
| MEE3 25 g/hl | 0 | 0 | 0 | 60 | 180 |
| MEE3 50 g/hl | 0 | 0 | 0 | 0 | 180 |

On constate que la précipitation de bitartrate de potassium en milieu synthétique est inhibée parfaitement par l'acide métatartrique mais aussi par les mannoprotéines extraites par action enzymatique, même si l'efficacité, à dose identique, est légèrement inférieure.

### Essai avec un vin blanc

Le tableau suivant montre les résultats obtenus avec l'acide métatartrique, les mannoprotéines extraites à la chaleur et les mannoprotéines extraites par digestion enzymatique.

| | | | | | |
|---|---|---|---|---|---|
| THK mg/100 ml | 0 | 50 | 75 | 100 | 125 |
| Témoin | 0 | 20 | 40 | 60 | 80 |
| Ac méta 5 g/hl | 0 | 0 | 0 | 20 | 20 |
| Ac méta 10 g/hl | 0 | 0 | 0 | 0 | 0 |
| Ac méta 25 g/hl | 0 | 0 | 0 | 0 | 0 |
| MEC 10 g/hl | 0 | 20 | 40 | 40 | 60 |
| MEC 25 g/hl | 0 | 20 | 20 | 20 | 60 |
| MEC 50 g/hl | 0 | 0 | 40 | 40 | 40 |
| MEE1 10 g/hl | 0 | 20 | 20 | 20 | 20 |
| MEE1 25 g/hl | 0 | 0 | 0 | 0 | 0 |
| MEE1 50 g/hl | 0 | 0 | 0 | 0 | 0 |
| MEE2 10 g/hl | 0 | 0 | 0 | 20 | 60 |
| MEE2 25 g/hl | 0 | 0 | 0 | 0 | 20 |
| MEE2 50 g/hl | 0 | 0 | 0 | 0 | 20 |
| MEE3 10 g/hl | 0 | 0 | 0 | 20 | 20 |
| MEE3 25 g/hl | 0 | 0 | 0 | 0 | 40 |
| MEE3 50 g/hl | 0 | 0 | 0 | 0 | 40 |

On constate que l'échantillon témoin montre une précipitation tartrique dès l'ajout de 50 mg/l, ce qui est le signe d'un vin particulièrement instable.

L'acide métatartrique et l'une des mannoprotéines extraites par digestion enzymatique sont capables d'éviter la précipitation jusqu'à des ajouts de 125 g/hl.

Les autres mannoprotéines extraites par digestion enzymatique donnent également de très bons résultats puisque tout vin qui ne précipite pas avec une dose de 75 g/hl est considéré comme stable.

Les mannoprotéines extraites à la chaleur n'ont aucune efficacité même à fort dosage.

### 2.- Détermination de la tenue au froid.

Ce test de tenue au froid consiste à maintenir les échantillons au froid, par - 4° C, pendant 6 jours, après les avoir filtrés sur une membrane à porosité de 1 µm.

L'absence de cristallisation dans ces conditions permet de considérer le vin testé comme stable.

Les résultats donnés dans le tableau ci-dessous montrent les résultats obtenus sur différents vins blancs, rosés et rouges.

| | | | | |
|---|---|---|---|---|
| *** : cristallisation | | | | |
| ND : non déterminé | | | | |
| 0 : pas de cristallisation | | | | |

| **Vins** | **Témoin** | **Ac méta 10 g/hl** | **MEC 25 g/hl** | **MEE1 25 g/hl** |
|---|---|---|---|---|
| Blanc 1 | *** | 0 | *** | 0 |
| Blanc 2 | *** | 0 | *** | 0 |
| Blanc 3 | *** | ND | *** | 0 |
| Blanc 4 | *** | ND | *** | 0 |
| Blanc 5 | *** | ND | *** | 0 |
| Blanc 6 | *** | ND | *** | 0 |
| Rosé 1 | *** | ND | *** | 0 |
| Rosé 2 | *** | 0 | *** | 0 |
| Rouge 1 | *** | *** | *** | 0 |
| Rouge 2 | *** | *** | *** | 0 |
| Rouge 3 | *** | *** | *** | 0 |

On constate que les mannoprotéines extraites par digestion enzymatique de parois de levure empêchent la formation de cristaux avec une dose de 25 g/hl.

On peut encore confirmer le résultat visuel en déterminant la différence de concentration en potassium des vins en mg/l avant et après passage au froid, comme présenté dans les tableaux suivants.

| Résultats obtenus avec le vin blanc N° 3 | | | |
|---|---|---|---|
| **Mannoprotéines** | **0 g/hl** | **15 g/hl** | **25 g/hl** |
| MEC | 400 | 350 | 150 |
| MEE1 | 400 | 250 | 0 |
| MEE2 | 400 | 200 | 0 |
| MEE3 | 400 | 300 | 0 |

| Résultats obtenus avec le vin rosé N° 1 | | |
|---|---|---|
| **Différentes modalités** | **Potassium mg/l** | **Ac Tartrique mg/l** |
| Témoin | 80 | 150 |
| MEC 25 g/hl | 30 | 50 |
| MEE1 25 g/hl | 0 | 0 |

Résultats obtenus avec les vins rouges N° 1, 2 et 3 correspondant à un vin rouge non collé, à un vin rouge collé à la gélatine à 10 g/hl et à un vin rouge collé à l'albumine d'oeuf à 10 g/hl.

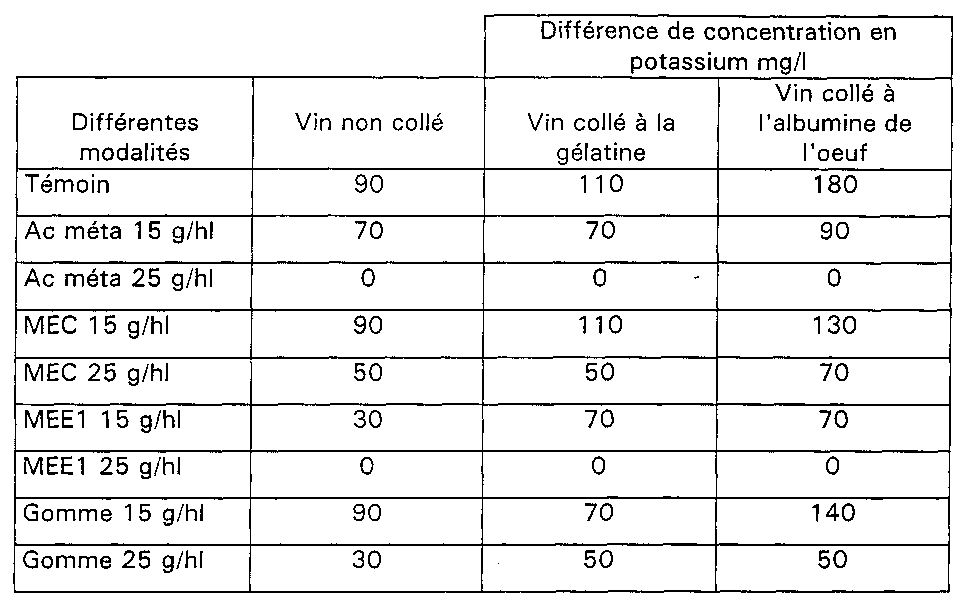

On constate que l'acide métatartrique donne de bons résultats à partir de 25 g/hl.

Les mannoprotéines extraites par digestion enzymatique ont aussi une excellente efficacité à la dose de 25 g/hl, ce qui est également la concentration qui permet de stabiliser parfaitement les trois vins blancs, rosés et rouges.

Les mannoprotéines extraites à la chaleur et la gomme arabique, avec des doses acceptables, n'inhibent pas en totalité la précipitation tartrique.

### 3.- Durée de l'efficacité

Des essais ont permis de comparer l'efficacité de l'acide métatartrique et des mannoprotéines obtenues par digestion enzymatique.

Le test consiste à placer pendant 10 semaines à 30° C un échantillon traité, puis à l'exposer au froid. Le dosage du potassium avant et après passage au froid permet de constater la stabilité tartrique du vin traité avec l'extrait (MEE1) et l'instabilité du vin témoin ou du vin traité à l'acide métatartrique. En effet, lors du séjour à 30° C, l'acide métatartrique s'hydrolyse et perd son pouvoir protecteur : de plus, il libère de l'acide tartrique qui augmente l'état de sursaturation du vin et favorise même la cristallisation du tartre.

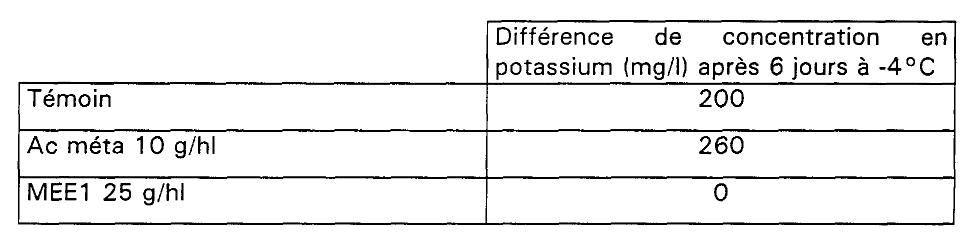

### II/ STABILISATION PROTEIQUE

La stabilité protéique des vins est appréciée par un test dit "à la chaleur" qui consiste à soumettre le vin à une température de 80°C pendant 30 mn. On mesure la turbidité par néphélométrie, exprimée en unités NTU. On corrèle la dose de bentonite nécessaire pour que la mesure de turbidité reste inférieure à 2 NTU.

Le tableau ci-dessous montre les résultats obtenus pour trois vins blancs traités par les différentes mannoprotéines.

| **Différentes modalités** | **Turbidité NTU** | **Dose de bentonite g/hl** |
|---|---|---|
| Témoin vin 1 | 12 | 80 |
| Vin 1 + MEC 25 g/hl | 12 | 80 |
| Vin 1 +MEE1 25 g/hl | 4,4 | 30 |
| Vin 1 + MEE2 25 g/hl | 4,2 | 30 |
| Vin 1 + MEE3 25 g/hl | 4,3 | 30 |
| Témoin vin 2 | 23,1 | 120 |
| Vin 2 + MEC 25 g/hl | 23,4 | 120 |
| Vin 2 + MEE1 25 g/hl | 10,5 | 60 |
| Vin 2 + MEE2 25 g/hl | 10 | 60 |
| Témoin vin 3 | 13,8 | 90 |
| Vin 3 + MEC 25 g/hl | 14 | 90 |
| Vin 3 + MEE1 25 g/hl | 6,2 | 50 |
| Vin 3 + MEE3 25 g/hl | 5,8 | 50 |

Les résultats montrent bien, pour les mannoprotéines extraites par digestion enzymatique, la diminution de la quantité de bentonite nécessaire pour obtenir la stabilité des vins. La diminution de la dose de bentonite est de 50 %.

Ainsi les qualités organoleptiques sont moins appauvries et surtout les vins sont stabilisés de façon durable sans modification du goût puisque les mannoprotéines extraites par digestion enzymatique sont neutres de goût.

Les essais menés montrent tout l'intérêt présenté par les mannoprotéines extraites par digestion enzymatique, tant sur l'inhibition des sels tartriques que sur la stabilisation protéique des vins blancs, ceci avec de faibles doses.

Il convient maintenant d'étudier plus avant les mannoprotéines extraites par digestion enzymatique pour montrer la fraction susceptible d'être la plus efficace tant vis à vis des sels tartriques que de la stabilité protéique.

Préalablement, on peut comparer directement la composition des préparations de mannoprotéines extraites à la chaleur et par voie enzymatique. On constate dans le tableau ci-après que les mannoprotéines obtenues par digestion enzymatique possèdent une teneur en protéines nettement supérieure.

| mannoprotéines | % de protéines | % de polysaccharides | % de mannose | % de glucose |
|---|---|---|---|---|
| extraites par la chaleur | 4,2 | 93,8 | 92 | 8 |
| extraites enzymatiquement | **15** | 83,2 | 100 | 0 |

Les teneurs en protéines sont déterminées par la méthode de BRADFORD (1976). Les teneurs en polysaccharides par la méthode au phénol sulfurique (MONTREUIL et SPIK, 1963).

La composition osidique de la partie polysaccharidique est déterminée par chromatographie en phase gazeuse des monosaccharides libérés par hydrolyse à l'acide trifluoroacétique et dérivés par silylation (LLAUBERES, 1988).

Les préparations de mannoprotéines extraites à la chaleur (MEC) et de mannoprotéines extraites par voie enzymatique (MEE) sont analysées en détail par électrophorèse sur gel de polyacrylamide en conditions dénaturantes (SDS PAGE) permettant un tamisage moléculaire dont le résultat est donné dans le tableau ci-dessous.

| **Masses moléculaires en KDa (kilo Daltons)** | |
|---|---|
| MEC | MEE |
| 77,8 | 77,8 |
| 70 | |
| 44,1 | 44,1 |
| | 41,6 |
| 35,2 | 35,2 |
| 31,8 | 31,8 |
| | 30,3 |
| 27,5 | 27,5 |
| 25,2 | 25,2 |
| 23,2 | 23,2 |
| 21,3 | 21,3 |
| 19,8 | 19,8 |
| 18,4 | 18,4 |
| 17,2 | 17,2 |
| 16 | 16 |
| 15,2 | 15,2 |

On constate l'absence de la protéine de 70 KDa dans les MEE et l'absence des protéines de 30,3 KDa et 41,6 KDa dans le MEC.

On isole alors les protéines particulières dans les MEE par fractionnement.

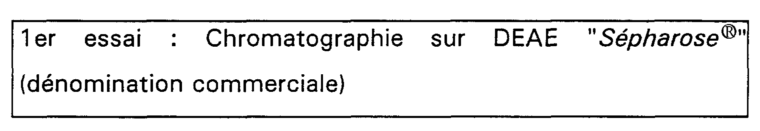

L'extrait brut de MEE (100 mg) est solubilisé dans 1 ml de tampon phosphate pH 8,0.

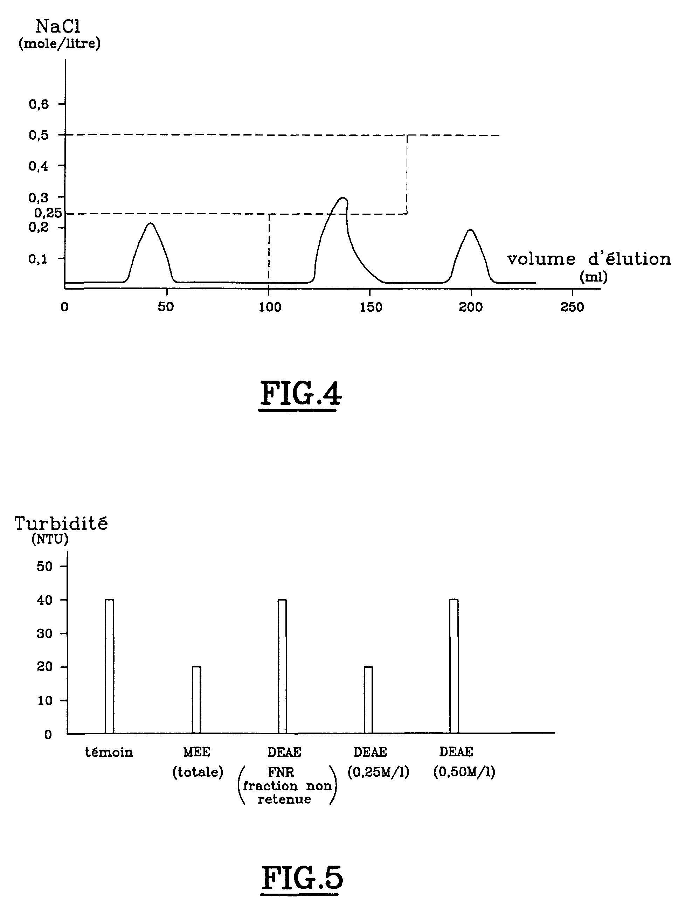

La colonne DEAE est lavée puis éluée par palier de NaCI 0,25 Mole/l puis 0,5 Mole/l. Des fractions de 3 ml sont recueillies puis les protéines sont détectées par mesure de l'absorption à 280 nm. Les fractions correspondant à chacun des trois pics sont assemblées, dialysées contre de l'eau et lyophilisées (voir figure 4).

On additionne 25 g/hl à un vin qui est ensuite soumis à un test à la chaleur pour déterminer le pouvoir de stabilisation protéique. On mesure la turbidité en NTU. Le résultat est représenté sur la figure 5.

On remarque le pouvoir de stabilisation protéique de la fraction éluée avec 0,25 M/I de NaCI et le faible effet de la fraction non retenue (FNR) et de la fraction à 0,50 M/I.

On a pu déterminer la teneur en protéines et en polysaccharides des MEE et de chacune des fractions éluées.

Comme on peut le constater dans le tableau suivant, la fraction active à 0,25 M/I renferme 16% de protéine, 78% de polysaccharide et son rendement d'extraction est de 60%.

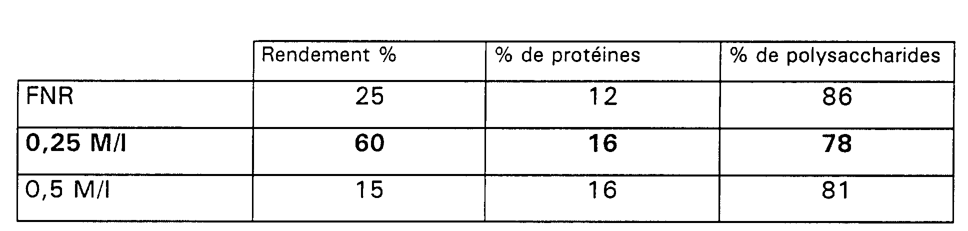

On cherche donc à purifier la fraction 0,25 M/I de NaCl par chromatographie d'affinité à l'aide de la concanavaline A (Con A), lectine qui se lie de façon réversible à des molécules comportant des résidus α - D mannopyranosyl et α - D - glucopyranosyl.

On peut ainsi séparer les protéines et les mannoprotéines de cette fraction spécifique 0,25 M/I de NaCI.

L'extrait 0,25 M/l de NaCI (60 mg) est solubilisé dans 1 ml de solution tampon citrate pH 5 et déposé sur la colonne de Con A *"Sépharose".*

Après lavage à la solution tampon pour éluer les protéines, on la soumet à une solution α D-mannoside à 0,5 M/I.

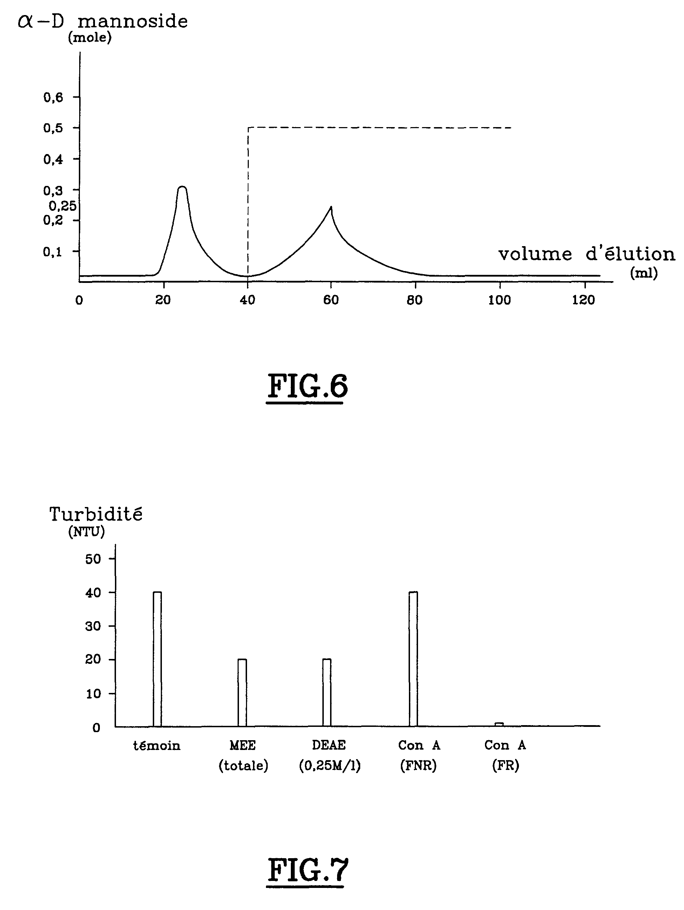

Les mannoprotéines sont éluées du gel. Des fractions de 3 ml sont recueillis et l'analyse par absorption à 280 nm donne les résultats donnés par la courbe de la figure 6.

Les fractions correspondant à chaque pic sont assemblés, dialysés contre de l'eau et lyophilisées.

On additionne chacune des fractions pour une dose de 25 g/hl.

Le test à la chaleur donne les résultats de la figure 7.

La fraction retenue est éluée par l'α-D-mannoside 0,5 M/I.

Cette fraction et les autres sont dosées en protéines et polysaccharides.

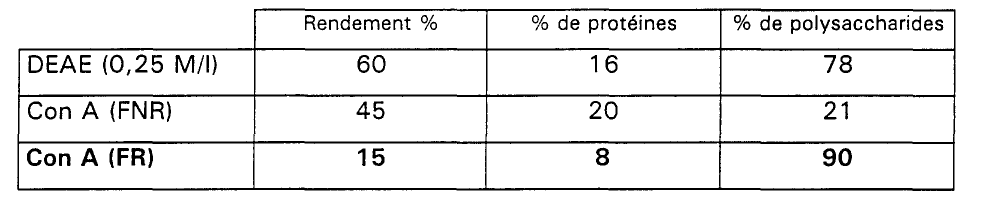

La fraction active représente 15% des MEE.

Cette fraction comprend 8% de protéines et 90% de mannose.

On peut réaliser comme précédemment une électrophorèse sur gel (SDS PAGE) qui montre que la mannoprotéine responsable de la stabilité protéique des vins blancs a une masse moléculaire de 31,8 KDa ou MP 32. C'est la seule protéine dont la concentration augmente au cours de la purification.

| **Masses moléculaires KDa** | | |
|---|---|---|
| MEE | DEAE (0,25 M/I) | Con A (FR) |
| 77,8 | 77,8 | |
| | 53 | |
| 44,1 | 44,1 | |
| 41,6 | | |
| 35,2 | 35,2 | |
| 31,8 | 31,8 | 31,8 |
| 30,3 | | |
| 27,5 | | |
| 25,2 | | |
| 23,2 | | |
| 21,3 | | |
| 19,8 | 19,8 | 19,8 |
| 18,4 | 18,4 | |
| 17,2 | 17,2 | 17,2 |
| 16 | 16 | 16 |
| 15,2 | 15,2 | 15,2 |

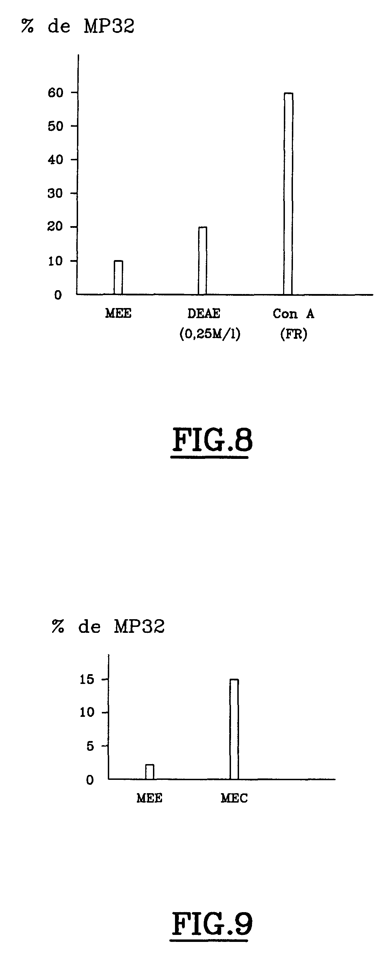

On a confirmation par électrophorèse capillaire que la MP 32 est présente à 2% dans les MEC et à 14% dans les MEE ; voir figure 9.

En ce qui concerne la stabilisation tartrique, on sépare les mannoprotéines par chromatographie liquide haute pression de tamisage moléculaire suivant leur taille en deux fractions qui sont dialysées contre de l'eau et lyophilisées.

Les fractions obtenues P1 et P2 sont additionnées à un vin blanc avec différentes doses. Les vins traités sont soumis à un test au froid et la concentration en potassium permet d'estimer la cristallisation tartrique.

On remarque après analyse que les MEE inhibent la cristallisation du tartrate de potassium dès 15 g/hl. La première fraction P1 n'arrive pas à inhiber cette cristallisation, par contre la fraction P2 permet une stabilisation tartrique à la dose de 5 g/hl.

L'analyse des protéines et polysaccharides contenues dans les différentes fractions donnent les résultants suivants :

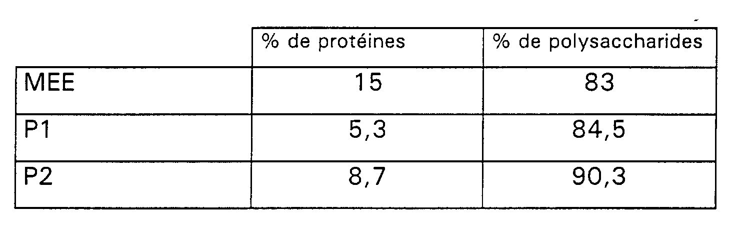

La fraction P2 qui permet une stabilisation tartrique est purifiée par chromatographie d'affinité grâce à la concanavaline (con A) en deux fractions l'une fraction retenue (FR) et l'autre, fraction non retenue (FNR) sur la lectine sont recueillies, dialysées et lyophilisées.

Après addition à différentes doses dans un vin, on vérifie que la fraction non retenue (FNR) ne permet pas d'inhiber la cristallisation des sels de l'acide tartrique.

La fraction retenue (FR) permet, elle, de prévenir la cristallisation pour une dose de 1,25 g/hl.

On peut à nouveau analyser la composition des fractions actives.

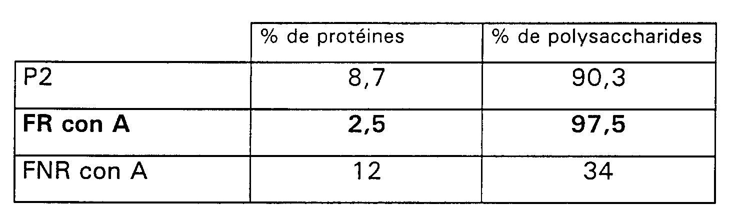

Ainsi la fraction active comprend 2,5 % de protéines et 97,5 % de polysaccharides.

Il convient alors de déterminer par électrophorèse sur gel les produits moléculaires des fractions purifiées.

| **Masses moléculaires en KDa (kilo Daltons)** | | | |
|---|---|---|---|
| MEE | P1 | P2 | FR con A |
| 77,8 | 77,8 | | |
| | | 53,3 | |
| 44,1 | 44,1 | | |
| 41,6 | | 41,6 | 41,6 |
| 35,2 | | 35,2 | |
| 31,8 | 31,8 | 31,8 | 31,8 |
| 30,3 | 30,3 | 30,3 | |
| 27,5 | 27,5 | 27,5 | |
| 25,2 | 25,2 | 25,2 | |
| 23,2 | | 23,2 | |
| 21,3 | | 21,3 | |
| 19,8 | | 19,8 | |
| 18,4 | | 18,4 | |
| 17,2 | 17,2 | 17,2 | 17,2 |
| 16 | 16 | 16 | |
| 15,2 | 15,2 | 15,2 | 15,2 |

La fraction active ne contient donc que quatre mannoprotéines dont les masses moléculaires sont 41,6 ; 31,8 ; 17,2 ; 15,2 KDa

La seule protéine dont la concentration augmente est la 41,6 KDa. C'est donc la mannoprotéine responsable de la stabilisation tartrique.

Or cette molécule est extractible uniquement et exclusivement de la paroi des levures par un complexe β-1-3 et β-1-6 glucanases.

Ceci permet d'expliquer pourquoi les mannoprotéines extraites par digestion enzymatique ont cette double faculté stabilisatrice vis à vis des vins et pourquoi elle présente un si grand intérêt, étant rappelé que les produits mis en oeuvre dans ce procédé sont déjà agréés auprès des organismes chargés de ce secteur alimentaire.

## Revendications

1. Traitement d'un vin en vue de sa stabilisation vis à vis des sels tartriques et des protéines, caractérisé en ce qu'il consiste à ajouter au vin des mannoprotéines extraites des parois de levures par digestion enzymatique par un mélange de β-1-3 et β-1-6 glucanases

2. Traitement d'un vin selon la revendication 1, caractérisé en ce que la levure appartient à l'espèce *Saccharomyces cerevisiae.*

3. Traitement selon la revendication 1 ou 2, caractérisé en ce que la quantité de mannoprotéines utilisée dans le vin est inférieure à 30 g/hl.

4. Procédé d'extraction de mannoprotéines par digestion enzymatique de levures pour la mise en oeuvre du traitement selon l'une quelconque des revendications 1 à 3, caractérisé en ce que :
• on fait incuber des parois de levure en milieu aqueux en présence de β-1-3 et du β-1-6 glucanases,
• on sépare les matières solides,
• on concentre la phase liquide, notamment par ultrafiltration.

5. Procédé d'extraction de mannoprotéines par digestion enzymatique de levures pour la mise en oeuvre du traitement selon la revendication 4, caractérisé en ce qu'une étape supplémentaire consiste à sécher le produit obtenu notamment par lyophilisation ou atomisation, par exemple dans un but de manipulation aisée.

6. Mannoprotéine obtenue par le procédé selon la revendication 4 ou 5 pour la mise en oeuvre du traitement selon les revendications 1 à 3, caractérisée en ce que l'analyse par chromatographie liquide à haute pression de tamisage moléculaire montre la présence d'un pic caractéristique (Y), en ce que l'analyse par électrophorèse SDS PAGE montre la présence de deux protéines de 41 600 et 31 800 Daltons et en ce que l'analyse par électrophorèse capillaire montre la présence d'un pic caractéristique correspondant à la mannoprotéine de 31 800 Daltons.

## Claims

1. Treatment of a wine with a view to its stabilisation vis-à-vis tartaric salts and proteins, characterised in that it comprises adding to the wine mannoproteins extracted from the walls of yeasts by enzymatic digestion by means of a mixture of β-1-3 and β-1-6 glucanases.

2. Treatment of a wine according to Claim 1, characterised in that the yeast belongs to the species Saccharomyces cerevisiae.

3. Treatment according to Claim 1 or 2, characterised in that the quantity of mannoproteins used in the wine is less than 30 g/hl.

4. Method of extracting mannoproteins by enzymatic digestion of yeasts for implementing the treatment according to any one of Claims 1 to 3, characterised in that:
• yeast walls are caused to incubate in an aqueous medium in the presence of β-1-3 and β-1-6 glucanases,
• the solid materials are separated,
• the liquid phase is concentrated, notably by ultrafiltration.

5. Method of extracting mannoproteins by enzymatic digestion of yeasts for implementing the treatment according to Claim 4, characterised in that a supplementary step comprises drying the product obtained, notably by freeze drying or atomisation, for example for the purpose of easy handling.

6. Mannoprotein obtained by the method according to Claim 4 or 5 for implementing the treatment according to Claims 1 to 3, characterised in that analysis by high-pressure liquid chromatography of molecular sieving shows the presence of a characteristic peak (Y), in that analysis by SDS PAGE electrophoresis shows the presence of two proteins of 41,600 and 31,800 daltons and in that analysis by capillary electrophoresis shows the presence of a characteristic peak corresponding to the mannoprotein of 31,800 daltons.

## Patentansprüche

1. Behandlung eines Weins im Hinblick auf seine Stabilisierung gegenüber Tatrat-Salzen und Proteinen, dadurch gekennzeichnet, daß sie darin besteht, dem Wein Mannoproteine zuzufügen, die aus Hefewänden durch enzymatische Digestion mittels einer Mischung aus β-1-3- und β-1-6-Glukanasen extrahiert werden.

2. Behandlung eines Weins nach Anspruch 1, dadurch gekennzeichnet, daß die Hefe zur Gattung der *Saccharomyzene cerevisiae* gehört.

3. Behandlung nach Anspruch 1 oder 2, dadurch gekennzeichnet, daß die im Wein verwendete Menge der Mannoproteine kleiner als 30 g/hl ist.

4. Verfahren zum Extrahieren von Mannoproteinen durch enzymatische Digestion von Hefen für die Ausführung der Behandlung nach einem der Ansprüche 1 bis 3, dadurch gekennzeichnet, daß:
- die Hefewände in wäßriger Umgebung in Gegenwart von β-1-3- und β-1-6 Glukanasen inkubiert werden,
- die Festkörperstoffe getrennt werden,
- die flüssige Phase insbesondere durch Ultrafiltrierung konzentriert wird.

5. Verfahren zum Extrahieren von Mannoproteinen durch enzymatische Digestion von Hefen für die Ausführung der Behandlung nach Anspruch 4, dadurch gekennzeichnet, daß ein zusätzlicher Schritt darin besteht, das erhaltene Produkt insbesondere durch Gefriertrocknung oder Zerstäubung zu trocken, um beispielsweise die Handhabung zu erleichtern.

6. Mannoprotein, das durch das Verfahren nach Anspruch 4 oder 5 erhalten wird, für die Ausführung der Behandlung nach den Ansprüchen 1 bis 3, dadurch gekennzeichnet, daß die Analyse durch Flüssigkeitschromatographie bei hohem Druck mit molekularer Siebung das Vorhandensein einer charakteristischen Spitze (Y) zeigt, daß die Analyse durch SDS-PAGE-Elektrophorese das Vorhandensein zweier Proteine mit 41600 und 31800 Daltons zeigt und daß die Analyse durch Kapillar-Elektrophorese das Vorhandensein einer charakteristischen Spitze, die dem Mannoprotein mit 31800 Daltons entspricht, zeigt.
